# EUROPÄISCHE PATENTSCHRIFT

(11) **EP 0 531 331 B1**
(45) Veröffentlichungstag und Bekanntmachung des Hinweises auf die Patenterteilung: **26.10.1994**
(21) Anmeldenummer: 91909384.9
(22) Anmeldetag: 21.05.1991
(51) Int. Cl.: H01S 3/09, A61F 9/00, H02J 7/34

(54) **LASERSYSTEM**
LASER SYSTEM
SYSTEME LASER

(30) Priorität: 30.05.1990 DE 4017441
(43) Veröffentlichungstag der Anmeldung: 17.03.1993
(73) Patentinhaber: WEIMEL, Erich, D-91220 Schnaittach (DE)
(72) Erfinder: WEIMEL, Erich, D-91220 Schnaittach (DE)
(74) Vertreter: Münich, Wilhelm, Dr.
(86) Internationale Anmeldenummer: DE9100418
(87) Internationale Veröffentlichungsnummer: WO9119337

(56) Entgegenhaltungen:
- EP-A- 0 024 268
- EP-A- 0 230 095
- US-A- 3 453 490
- US-A- 4 430 739

## Beschreibung

### Technisches Gebiet

Die Erfindung bezieht sich auf ein Lasersystem mit einem Laser, der zwischen mindestens zwei Leistungsstufen umschaltbar ist, und einer Energieversorgungseinheit für den Laser, an der als Versorgungs-Eingangsspannung Netzspannung anliegt.

Derartige Lasersysteme sind allgemein bekannt: Beispielsweise werden Argon- oder Krypton-Laser mit wenigstens zwei Leistungsstufen in der Ophthalmologie zur Koagulation des Augenhintergrundes eingesetzt (EP-A-0 230 095).

### Stand der Technik

Die niedrige Leistungsstufe ist bei den bekannten Lasern so ausgelegt, daß der Laserstrahl zwar auf dem Augenhintergrund wahrgenommen werden kann, aber keine Koagulationswirkung hervorruft; damit kann der Laser auf den zu behandelnden Bereich ausgerichtet werden. Durch Schalten des Lasers auf hohe Leistung wird dann die gewünschte Behandlung des Augenhintergrundes erreicht.

Nun weisen insbesondere Argon-Laser einen vergleichsweise schlechten Wirkungsgrad auf. Damit muß die Energieversorgungseinheit für den Laser so ausgelegt sein, daß sie eine vergleichsweise hohe Maximalleistung hat, - typischerweise 5 kW bis 10 kW - um den Laser mit einer therapeutisch wirksamen Leistung betreiben zu können. Energieversorgungseinheiten mit derartigen Maximalleistungen können aber nicht mehr an das "normale" Stromnetz angeschlossen werden, da üblicherweise Steckdosen für 220V Wechselstrom nur mit 16 A abgesichert sind, so daß keine größere Leistung als ca 3,5 kW entnommen werden kann. Darüberhinaus sind viele der handelsüblichen Energieversorgungseinheiten für Argon-Laser mit einer therapeutisch wirksamen Leistung wassergekühlt.

Dies bedeutet in der Regel, daß Argon-Behandlungslaser in Praxis-Räumen nicht ohne besondere Installationsmaßnahmen - Drehstromanschluß, Wasseranschluß etc. - aufgestellt werden können.

### Darstellung der Erfindung

Der Erfindung liegt die Aufgabe zugrunde, ein Lasersystem gemäß dem Oberbegriff des Anspruchs 1 und insbesondere einen Argon-Behandlungslaser beispielsweise für ophthalmologische Anwendungen derart weiterzubilden, daß die Energieversorgungseinheit des Lasersystems eine maximale Leistungsaufnahme hat, die eine Versorgung aus Norm-Hausinstallations-Stromnetzen zuläßt.

Eine erfindungsgemäße Lösung dieser Aufgabe ist im Anspruch 1 angegeben. Weiterbildungen der Erfindung sind Gegenstand der Unteransprüche.

Die Erfindung geht von der Erkenntnis aus, daß gattungsgemäße Lasersysteme in der Regel nur für einen verhältnismäßig kurzen Zeitraum mit hoher Leistung betrieben werden. Beispielsweise bei Argon-Lasersystemen zur Behandlung des Augenhintergrundes liegt die Dauer der therapeutisch wirksamen "Laserschüsse" im Sekundenbereich und darunter, wobei zwischen den einzelnen "Laserschüssen" oft mehrere Minuten vergehen, in denen der Arzt die Ausrichtung des Augenhintergrundes und/oder den Behandlungserfolg überprüft.

Deshalb wird erfindungsgemäß die Energieversorgungseinheit so ausgelegt, daß sie die für therapeutisch wirksame "Laserpulse" erforderliche Maximalleistung nicht bzw. nicht ausschließlich dem Stromnetz, sondern - zum Teil - einem Energiespeicher entnimmt, der in dem Zeitraum, in dem der Laser nicht bzw. mit niedriger Leistung arbeitet, wieder aufgeladen wird.

Hierzu weist die Energieversorgungseinheit eine Verteilereinheit auf, an deren Eingangsanschluß die Netzspannung anliegt, und die mit einem Lasernetzteil und einer Ladeeinheit für eine Akkumulatoreinheit derart verbunden ist, daß während des Zeitraum, während dem der Laser mit niedriger Leistung betrieben wird, zusätzlich zum Lasernetzteil auch die Ladeeinheit aus dem Stromnetz mit Energie versorgt wird, und daß während des Zeitraums, während dem der Laser mit hoher Leistung betrieben wird, der Ausgangsanschluß der Akkumulatoreinheit mit dem Lasernetzteil verbunden ist.

Durch im Anspruch 2 gekennzeichnete Weiterbildung, bei der während des Zeitraums, während dem der Laser mit hoher Leistung betrieben wird, die Verteilereinheit die Verbindung Stromnetz/Akkumulatoreinheit unterbricht, kann die gesamte aus dem Stromnetz entnehmbare Leistung zur Versorgung des Lasers verwendet werden, so daß die Akkumulatoreinheit nur die Differenz zwischen der zur Erzeugung therapeutisch wirksamer Laserpulse erforderlichen "Spitzenleistung" und der aus dem Stromnetz entnehmbaren Maximalleistung bereitstellen muß.

Im Anspruch 3 ist eine Ausgestaltung der Erfindung angegeben, bei der das Lasernetzteil ein DC/DC-Schaltnetzteil und die Ladeeinheit eine DC-Ladeeinheit ist. Hierdurch ist es möglich, in der Energieversorgungseinheit nach der Verteilereinheit mit Gleichstrom "zu arbeiten", so daß keine Wandlung der Akkumulator-Ausgangsspannung in Wechselstrom erforderlich ist.

Eventuell erforderliche "Anpassungen" können dabei leicht dadurch ausgeführt werden, daß zwischen den Ausgangsanschluß der Akkumulatoreinheit und einen Eingangsanschluß des Lasernetzteils ein Spannungswandler geschaltet ist (Anspruch 4).

Das erfindungsgemäße Konzept zur Bereitstellung der für hohe Leistungen bei schaltbaren Lasern erforderlichen Zusatzleistung kann bei beliebigen Lasersystemen eingesetzt werden.

Von besonderem Vorteil ist das erfindungsgemäße Konzept jedoch bei Argon-Lasersystemen, die zu therapeutischen Zwecken eingesetzt werden, wie beispielsweise zur Koagulation des Augenhintergrundes (Anspruch 5). Erfindungsgemäß ausgebildete Laser können ohne besondere "Installationsmaßnahmen" einfach an normale Steckdosen, die in üblicher Weise abgesichert sind, angeschlossen werden, und benötigen insbesondere keine Wasserkühlung für die Energieversorgungseinheit.

Dabei ist es nicht erforderlich, daß die niedrige Leistungsstufe, in der der Laser dauernd aus dem Stromnetz mit Energie versorgt wird, eine Leistungsstufe ist, in der der Laser einen Laserstrahl abgibt. Vielmehr ist die erfindungsgemäße Ausbildung gerade bei einem Lasersystem von Vorteil, bei dem die niedrige Leistungsstufe ein Stand-by-Modus ist, in der die Laserröhre unterhalb der Laserschwelle betrieben wird (Anspruch 6). Ein derartiges System ist in der Anmeldung WO-A-91 193 336 desselben Anmelders beschrieben : Bei diesem System wird der Strahl des Argon-Lasers nicht zum Ausrichten benutzt. Vielmehr ist koaxial mit dem Weg des Argon-Laserstrahls der Strahl eines Zielstrahllasers, beispielsweise eines Helium-Neon-Lasers, der einen guten Wirkungsgrad und damit eine geringe Leistungsaufnahme hat, geführt; der Helium-Neon-Laserstrahl wird zum Ausrichten des Argon-Lasers benutzt. Der Argon-Laser wird dabei in einem sogenannten Stand-by-Modus unterhalb der Laserschwelle betrieben, so daß ein therapeutisch wirksamer Laserstrahl ohne Verzögerung ausgelöst werden kann. In dem Stand-by-Modus ist die Leistungsaufnahme der Laserröhre besonders gering, so daß in kurzer Speicher der Akkumulator aufgeladen werden kann.

### Kurze Beschreibung der Zeichnung

Die Erfindung wird nachstehend ohne Beschränkung des allgemeinen Erfindungsgedankens anhand von Ausführungsbeispielen unter Bezugnahme auf die Zeichnung exemplarisch beschrieben, auf die im übrigen bezüglich der Offenbarung aller im Text nicht näher erläuterten erfindungsgemäßen Einzelheiten ausdrücklich verwiesen wird. Es zeigen:

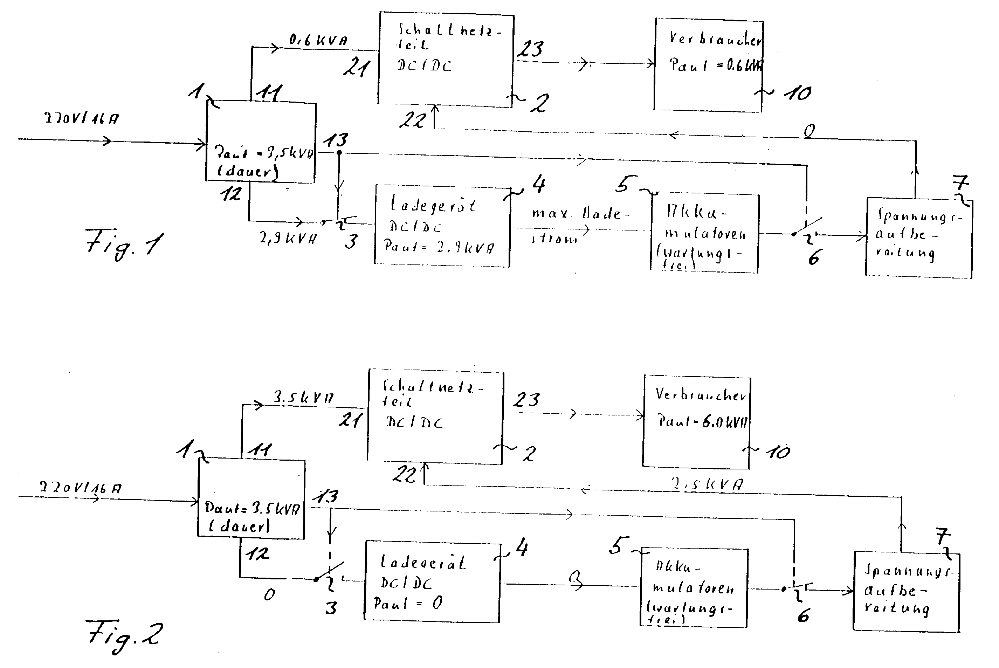

- Fig. 1 und 2: die Energieversorgungseinheit eines erfindungsgemäßen Lasersystems in unterschiedlichen Betriebszuständen.

### Darstellung eines Ausführungsbeispiels

In den Figuren ist schematisch die Energieversorgungseinheit eines erfindungsgemäßen Lasersystems dargestellt. Der eigentliche Laser mit Laserröhre, Laserspiegeln und Pumpeinheit, der typischerweise ein Dauerstrich-Argon-Laser für ophthalmologische Behandlungen ist, ist lediglich schematisch als Verbraucher 10 bezeichnet.

Die Energieversorgungseinheit ist so ausgelegt, daß sie an einer üblichen mit 16 A abgesicherten 220 V-Steckdose betrieben werden kann. Die Wechselspannung liegt an einer Verteilereinheit 1 an, die zwei Last-Ausgangsanschlüsse 11 und 12 und einen Steuer-Ausgangsanschluß 13 hat. In der Verteilereinheit erfolgt bevorzugt eine AC/DC-Wandlung, so daß an den Ausgangsanschlüssen 11 und 12 eine Spannung ansteht, die in DC/DC-Schaltnetzteilen "weiter verarbeitet" werden kann.

Der Ausgangsanschluß 11 der Verteilereinheit 1 ist mit einem Eingangsanschluß 21 eines DC/DC-Schaltnetzteils 2 verbunden, dessen Ausgangsanschluß 23 mit dem bereits erwähnten eigentlichen Laser 10 verbunden ist. Der andere Ausgangsanschluß 12 ist über einen Schalter 3 mit dem Eingangsanschluß einer Ladeeinheit 4 für eine Akkumulatoreinheit 5 verbunden, deren Ausgangsanschluß über einen weiteren Schalter 6 mit einer Spannungsaufbereitungsschaltung 7 verbunden ist, die wiederum mit einem weiteren Eingangsanschluß 22 des Schaltnetzteils 2 verbunden ist. Die Akkumulatoreinheit ist eine aus handelsüblichen und insbesondere wartungsfreien Blei-, NiCd-Akkumulatoren oder dgl. aufgebaute Einheit, bei der die einzelnen Akkumulatoren in Reihe oder parallel geschaltet sind. Die Spannungsaufbereitungsschaltung 7 erfüllt im wesentlichen die Funktion, die Ausgangs-Gleichspannung der Akkumulatoreinheit 5 so aufzubereiten, daß sich eine für das Schaltnetzteil 2 als Eingangsspannung geeignete Schaltung ergibt.

Die Verteilereinheit 1 steuert ferner über den Steueranschluß 13 die Schalter 3 und 6.

Nachfolgend wird die Funktion der beschriebenen Energieversorgungseinheit erläutert:

Fig. 1 zeigt den Stromfluß im sogenannten Stand-by-Betrieb, bei dem die Laserröhre des Argon-Lasers 10 unterhalb der Laserschwelle betrieben wird. Der Laser gibt in dieser Betriebsart zwar keinen Laserstrahl ab, ist aber sofort betriebsbereit, wenn beispielsweise ein Arzt durch Drücken eines nicht dargestellten Fußschalters einen therapeutisch wirksamen Laserpuls auslösen will. In dieser Betriebsweise teilt die Verteilereinheit 1 die aus dem Stromnetz entnommene Leistung von 220 V * 16 A ≈ 3,5 kW so auf, daß 0,6 kW an das Schaltnetzteil 2 und 2,9 kW über den geschlossenen Schalter 3 an die Ladeeinheit 4 abgegeben werden. Da in dieser Betriebsweise der Schalter 6 geöffnet ist, lädt die Ladeeinheit 4 die Akkumulatoreinheit 5 solange, bis diese voll geladen ist.

Die an das Schaltnetzteil 2 abgebene Leistung genügt zwar nicht, um die Laserschwelle der Laserröhre zu erreichen, sie ist aber ausreichend, um die Laserröhre in einem Zustand zu halten, aus dem heraus sie ohne nennenswerte Verzögerung einen therapeutisch wirksamen Laserpuls abgeben kann.

Soll nun ein derartiger Laserpuls abgegeben werden, so öffnet die Verteilereinheit 2 den Schalter 3; damit liegt die gesamte, aus dem Stromnetz entnommene Leistung von 3,5 kV am Schaltnetzteil 2 an. Ferner schließt die Verteilereinheit 1 den Schalter 6, so daß auch die Ausgangsspannung der Akkumulatoreinheit 5 entsprechend aufbereitet am Schaltnetzteil 2 anliegt. Bei dem gezeigten Ausführungsbeispiel kann aus der Akkumulatoreinheit zumindest für die typische Dauer eines für ophthalmische Zwecke benötigten Laserpulses - der höchstens eine Breite von einigen Sekunden hat - eine Leistung von 2,5 kW entommen werden, so daß die Laserröhre insgesamt mit einer Leistung von 6 kW betrieben werden kann.

Vorstehend ist die Erfindung anhand eines Ausführungsbeispiels ohne Beschränkung des allgemeinen Erfindungsgedankens beschrieben worden. Beispielsweise können auch andere Laserröhren als Argon-Laserröhren verwendet werden. Auch ist es möglich, andere Komponenten, wie beispielsweise andere Netzteile anstelle von Schaltnetzteilen einzusetzen. Ferner ist die angegebene Dimensionierung nur beispielhaft zu verstehen. So ist es selbstverständlich erforderlich, beispielsweise für 110 V-Netze oder anders abgesicherte Netze andere Leistungsaufteilungen vorzunehmen.

## Patentansprüche

1. Lasersystem mit einem Laser, der zwischen mindestens zwei Leistungsstufen umschaltbar ist, und einer Energieversorgungseinheit für den Laser, an der als Versorgungs-Eingangsspannung-Netzspannung anliegt,
dadurch **gekennzeichnet**, daß die Energieversorgungseinheit eine Verteilereinheit (1) aufweist, an deren Eingangsanschluß die Netzspannung anliegt, und die mit einem Lasernetzteil (2) und einer Ladeeinheit (4) für eine Akkumulatoreinheit (5) derart verbunden ist, daß während des Zeitraum, während dem der Laser (10) mit niedriger Leistung betrieben wird, zusätzlich zum Lasernetzteil (2) auch die Ladeeinheit (4) aus dem Stromnetz mit Energie versorgt wird, und daß während des Zeitraums, während dem der Laser (10) mit hoher Leistung betrieben wird, der Ausgangsanschluß der Akkumulatoreinheit (5) mit dem Lasernetzteil (2) verbunden ist.

2. Lasersystem nach Anspruch 1,
dadurch **gekennzeichnet**, daß ein von der Verteilereinheit (1) gesteuerter Schalter (3) vorgesehen ist, der während des Zeitraums, während dem der Laser (10) mit hoher Leistung betrieben wird, die Verbindung Stromnetz/Akkumulatoreinheit (5) unterbricht.

3. Lasersystem nach Anspruch 1 oder 2,
dadurch **gekennzeichnet**, daß das Lasernetzteil (2) ein DC/DC-Schaltnetzteil und die Ladeeinheit (4) eine DC-Ladeeinheit ist.

4. Lasersystem nach Anspruch 3,
dadurch **gekennzeichnet**, daß zwischen den Ausgangsanschluß der Akkumulatoreinheit (5) und einen Eingangsanschluß (22) des Lasernetzteils (2) eine Spannungsaufbereitungseinheit (7) geschaltet ist.

5. Lasersystem nach einem der Ansprüche 1 bis 4,
dadurch **gekennzeichnet**, daß der Laser (10) ein Argon-Laser insbesondere für ophthalmologische Zwecke ist.

6. Lasersystem nach Anspruch 5,
dadurch **gekennzeichnet**, daß die niedrige Leistungsstufe ein Stand-by-Modus ist, in der die Laserröhre unterhalb der Laserschwelle betrieben wird.

## Claims

1. Laser system including a laser adapted to be switched between at least two power levels, and an energy supply means for the laser, to which the mains voltage is applied as supply input voltage,
**characterized** in that said energy supply means comprises a distributor means (1) having an input terminal which the mains voltage is applied to, and connected to a lower power-pack (2) and a charging means (4) for an accumulator unit (5) in such a way that during the period in which said laser (10) is operated at a low power level said charging means (4), in addition to said laser power-pack (2), is also supplied with energy from the supply mains, and that during the period in which said laser (10) is operated at a high power level the output terminal of said accumulator means (5) is connected to said laser power-pack (2).

2. Laser system according to Claim 1,
**characterized** in that a switch (3) controlled by said distributor means (1) is provided which interrupts the connection between the supply mains and said accumulator means (5) during the period in which said laser (10) is operated at a high power level.

3. Laser system according to Claim 1 or 2,
**characterized** in that said laser power-pack (2) is a d.c./d.c. switching power-pack and that said charging means (4) is a d.c. charging means.

4. Laser system according to Claim 3,
**characterized** in that a voltage processing means (7) is connected be tween the output terminal of said accumulator means (5) and an input terminal (22) of said laser power-pack (2).

5. Laser system according to any of Claims 1 to 4,
**characterized** in that said laser (10) is an argon laser, specifically for applications in ophthalmology.

6. Laser system according to Claim 5,
**characterized** in that the lower power level is a stand-by mode in which the laser tube is operated below the laser threshold.

## Revendications

1. Système laser comprenant un laser commutable entre au moins deux étages de puissance, ainsi qu'un module d'alimentation en énergie auquel la tension de réseau est appliquée comme tension d'alimentation d'entrée,
**caractérisé** en ce que le module d'alimentation en énergie comprend un dispositif distributeur (1), qui est prévu d'une connexion d'entrée à laquelle est appliquée la tension de réseau, et qui est relié à un bloc d'alimentation du laser (2) ainsi qu'à un dispositif de chargement (4) pour un module accumulateur (5), de façon qu'au cours de la période, dans laquelle le laser (10) est actif à un bas niveau de puissance, le dispositif de chargement (4), en outre que le bloc d'alimentation (2) du laser, est aussi alimenté en énergie emprunté du réseau d'alimentation, et qu'au cours de la période dans laquelle le laser est actif à un haut niveau de puissance, la connexion d'entrée dudit module accumulateur (5) est reliée au bloc d'alimentation (2) du laser.

2. Système laser selon la revendication 1,
**caractérisé** en ce qu'un commutateur (3) commandé par ledit dispositif distributeur (1) est prévu, qui coupe la connexion entre le réseau d'alimentation et ledit module accumulateur (5) au cours de la période dans laquelle ledit laser (10) est activé à un haut niveau de puissance.

3. Système laser selon la revendication 1 ou 2,
**caractérisé** en ce que ledit bloc d'alimentation du laser (2) est un bloc d'alimentation à commutation continu-continu, et en ce que ledit dispositif de chargement (4) est un dispositif de chargement à courant continu.

4. Système laser selon la revendication 3,
**caractérisé** en ce que des moyens de traitement de tension (7) sont branchés entre la connexion de sortie dudit module accumulateur (5) et une connexion d'entrée (22) dudit bloc d'alimentation (2) du laser.

5. Système laser selon une quelconque des revendications 1 à 4,
**caractérisé** en ce que ledit laser (10) est un laser à argon, en particulier pour des applications dans l'ophthalmologie.

6. Système laser selon la revendication 5,
**caractérisé** en ce que le niveau de puissance inférieur est un mode en état de service, où le tube laser est activé au dessous du seuil de l'effet laser.
